# EUROPÄISCHE PATENTANMELDUNG

(11) **EP 4 591 837 A1**
(43) Veröffentlichungstag der Anmeldung: **30.07.2025**
(21) Anmeldenummer: 24220300.8
(22) Anmeldetag: 16.12.2024
(51) Int. Cl.: A61F 2/95, A61F 2/966

(54) **VORRICHTUNG ZUM PLATZIEREN EINES STENTS**

(30) Priorität: 25.01.2024 DE 202024100371 U
(71) Anmelder: Bess Pro GmbH, 14167 Berlin (DE)
(72) Erfinder: SIEGNER, Georg, 12159 Berlin (DE); GREGOR, Michael, 12207 Berlin (DE)
(74) Vertreter: Griepenstroh, Jörg

(57) **Zusammenfassung**

Die Erfindung betrifft eine Vorrichtung 1 zum Platzieren eines Stents 2 in einem Gefäß oder einem Hohlorgan eines Menschen oder Tiers, aufweisend einen Applikator 3 und einen Stent 2 mit den folgenden Merkmalen:
a) Der Applikator 3 weist einen äußeren Tubus 7 und einen inneren Tubus 6 auf, wobei der innere Tubus 6 innerhalb des äußeren Tubus 7 geführt ist;
b) Der Stent 2 ist auf einem Stentträger 4 in einer Transportkartusche 5 angeordnet;
c) Der innere Tubus 6 weist an seinem distalen Ende ein erstes Kopplungselement 11 auf;
d) Das erste Kopplungselement 11 des Applikators 3 ist mit einem zweiten Kopplungselement 12 am proximalen Ende des Stentträgers 4 koppelbar;
e) Der Stentträger 4 ist im gekoppelten Zustand der Kopplungselemente 11,12 zusammen mit dem Stent 2 aus der Transportkartusche 5 heraus und in den äußeren Tubus 7 einziehbar um den Stent 2 innerhalb des äußeren Tubus 7 anzuordnen.

## Beschreibung

Die vorliegende Erfindung betrifft eine Vorrichtung zum Platzieren eines Stents in einem Gefäß oder einem Hohlorgan eines Menschen oder Tiers gemäß den Merkmalen des Anspruchs 1.

Es ist Stand der Technik, einen Stent mittels eines Applikators in einem Gefäß oder einem Hohlorgan eines Menschen oder Tiers zu platzieren. In der Regel werden einem Operateur Applikatoren mit bereits darin geladenen Stents zur Verfügung gestellt. Entsprechende Vorrichtungen bestehend aus dem Applikator mit geladenem Stent müssen vor ihrer Auslieferung sterilisiert werden. Da die Applikatoren in der Regel aus thermolabilen Kunststoffen hergestellt sind, wird ein Niedrigtemperatursterilisationsverfahren angewendet. Entsprechende Verfahren werden nur von wenigen dafür zugelassenen Dienstleistern durchgeführt.

Es besteht ein steigender Bedarf an patientenangepassten Stents. Dabei werden die Stents in ihrer Geometrie, Webart und Rückstellkraft angepasst, so dass sie für den konkreten Einzelfall möglichst wirksam und verträglich sind.

Problematisch ist, dass die patientenangepassten Stents nach ihrer Herstellung zwar vom Hersteller in den Applikator geladen werden können, die Vorrichtungen jedoch im Anschluss zunächst an den für die Niedrigtemperatursterilisation zugelassenen Dienstleister überreicht werden müssen. Die dafür erforderliche Transportzeit, als auch eine oftmals auftretende auslastungsbedingte Wartezeit beim Dienstleister führen zu erheblichen Verzögerungen bei der Auslieferung. Daher wird in der Praxis regelmäßig auf eine Anpassung der Stents verzichtet, insbesondere wenn der Eingriff für den Patienten zeitkritisch ist. Stattdessen wird auf Standardstents zurückgegriffen, was sich nachteilig auf die Passgenauigkeit, Wirksamkeit und Verträglichkeit des Stents auswirkt.

Hiervon ausgehend liegt der Erfindung die Aufgabe zugrunde, eine Vorrichtung zum Platzieren eines Stents in einem Gefäß oder einem Hohlorgan eines Menschen oder Tiers aufzuzeigen, bei welcher der Stent nicht mittels einer Niedrigtemperatursterilisation bei einem dafür zugelassenen Dienstleister sterilisiert werden muss.

Diese Aufgabe ist durch eine Vorrichtung zum Platzieren eines Stents mit den Merkmalen des Anspruchs 1 gelöst.

Vorteilhafte Weiterbildungen der Erfindung sind Gegenstand der Unteransprüche.

Die erfindungsgemäße Vorrichtung zum Platzieren eines Stents in einem Gefäß oder einem Hohlorgan eines Menschen oder Tiers weist einen Applikator und einen Stent auf. Der Applikator, auch Einführsystem genannt, weist einen äußeren Tubus und einen inneren Tubus auf. Dabei kann es sich insbesondere um einen äußeren Katheter und einen inneren Katheter handeln. Der innere Tubus ist innerhalb des äußeren Tubus geführt. Der innere Tubus und der äußere Tubus sind zueinander verlagerbar ausgebildet. Der Applikator ist bevorzugt mittels eines Niedrigtemperatursterilisationsverfahrens sterilisiert.

Der Stent ist auf einem Stentträger in einer Transportkartusche angeordnet. Insbesondere besteht der Stent aus Nitinol und ist selbstexpandierend, wobei der Stent in einem komprimierten Zustand in der Transportkartusche angeordnet ist. Der Stent kann eine Beschichtung aufweisen. Weiterhin bevorzugt ist der Stent thermostabil und für eine Dampfsterilisation geeignet. Der Stent kann somit samt Stentträger und Transportkartusche unabhängig vom Applikator sterilisiert werden, ohne dass dieser zu einem für Niedrigtemperatursterilisation zugelassenen Dienstleister überführt werden muss.

Der Applikator und der Stent werden einem Operateur getrennt voneinander zur Verfügung gestellt. Der Stent ist in diesem Ausgangszustand noch nicht im Applikator angeordnet, mithin noch nicht in diesem geladen.

Der innere Tubus weist an seinem distalen Ende ein erstes Kopplungselement auf. Distal bedeutet aus Operateurssicht weiter entfernt. Das erste Kopplungselement des Applikators ist mit einem zweiten Kopplungselement, welches am proximalen Ende des Stentträgers angeordnet ist, koppelbar. Proximal bedeutet aus Operationssicht näher gelegen. Der Stentträger kann somit vom Operateur mit dem Applikator verbunden werden.

Der Stentträger ist im gekoppelten Zustand der Kopplungselemente zusammen mit dem Stent aus der Kartusche heraus und in den äußeren Tubus einziehbar, um den Stent innerhalb des äußeren Tubus anzuordnen. Der Operateur kann den Stent selbstständig in dem Applikator platzieren bzw. in diesen laden. Durch die erfindungsgemäße Vorrichtung kann somit die zeitaufwendige Niedrigtemperatursterilisation bei einem dafür zugelassenen Dienstleister vermieden werden, da der Stent unabhängig vom Applikator direkt vom Hersteller sterilisiert werden kann. Die Kopplung zwischen Stent und Applikator erfolgt erfindungsgemäß nicht vor der Sterilisation, sondern wird vom Operateur selbst vor dem eigentlichen Eingriff vorgenommen. Ein patientenangepasster Stent kann folglich schneller zur Verfügung gestellt werden.

Vorzugsweise weist das erste Kopplungselement oder das zweite Kopplungselement ein Außengewinde auf, wobei das jeweils andere Kopplungselement ein Innengewinde aufweist. Der Stentträger und der innere Tubus des Applikators können mittels der Gewinde der Kopplungselemente miteinander verschraubt werden, so dass eine sichere Verbindung zwischen dem Stentträger und dem inneren Tubus hergestellt ist. Durch eine Drehung des inneren Tubus kann das Außengewinde mit dem Innengewinde gekoppelt und wieder gelöst werden.

Bevorzugt weist der Applikator an seinem proximalen Ende einen distalen Handgriff und einen proximalen Handgriff auf, wobei der distale Handgriff mit einem proximalen Ende des äußeren Tubus und der proximale Handgriff mit einem proximalen Ende des inneren Tubus gekoppelt ist. Mittels der Handgriffe ist der innere Tubus innerhalb des äußeren Tubus verlagerbar. Beim Ladevorgang wird der innere Tubus mit dem daran gekoppelten Stentträger samt Stent in proximaler Richtung in den äußeren Tubus hineingezogen. Um den Stent innerhalb des Gefäßes oder des Hohlorgans des Patienten zu platzieren, wird der innere Tubus und der mit diesem gekoppelte Stentträger samt Stent in distaler Richtung aus dem äußeren Tubus herausgeschoben.

In einer vorteilhaften Ausführungsvariante der Erfindung ist die Transportkartusche an ihrem proximalen Ende aufgeweitet. Besonders bevorzugt ist der Innendurchmesser der Transportkartusche im aufgeweiteten Bereich größer als der Außendurchmesser des äußeren Tubus, wobei der Innendurchmesser zumindest des an dem an den aufgeweiteten Bereich angrenzenden Bereich der Transportkartusche kleiner ist als der Außendurchmesser des äußeren Tubus. Diese Ausgestaltung ermöglicht einen einfachen Ladevorgang des Stentträgers samt Stent aus der Transportkartusche in den äußeren Tubus hinein.

Im gekoppelten Zustand befindet sich der innere Tubus zunächst innerhalb der Transportkartusche. Der äußere Tubus kann in distaler Richtung über den inneren Tubus vorgeschoben werden, bis er mit dem Bereich der Transportkartusche zur Anlage kommt, dessen Innendurchmesser kleiner ist als der Außendurchmesser des äußeren Tubus. Der innere Tubus kann anschließend nach proximal verlagert werden, so dass dieser zusammen mit dem gekoppelten Stentträger samt Stent in den äußeren Tubus hineingezogen wird. Der Stentträger samt Stent befindet sich nun innerhalb des äußeren Tubus und die Vorrichtung ist im geladenen Zustand. Die Transportkartusche kann abgezogen und entsorgt werden.

Die Transportkartusche ist bevorzugt an ihren jeweiligen Enden geöffnet. Auf der proximalen Seite ermöglicht dies eine einfache Kopplung des in der Transportkartusche befindlichen Stentträgers mit dem inneren Tubus, während die Öffnung auf der distalen Seite dafür sorgt, dass beim Laden des Stents in den Applikator kein Vakuum innerhalb der Transportkartusche entsteht, was eine entgegen der Laderichtung wirkende Kraft erzeugen würde.

Vorzugsweise ist der Stentträger an seinem proximalen Ende konisch geformt. Insbesondere ist das am proximalen Ende des Stentträgers angeordnete zweite Kopplungselement konisch geformt. Die konische Form ergibt sich durch einen in proximaler Richtung abnehmenden Außendurchmesser des proximalen Endes des Stentträger bzw. des zweiten Kopplungselements. Die konische Formgebung hat zwei Vorteile. Zum einen wird ein Verkanten des Stentträgers beim Einziehen in den äußeren Tubus vermieden. Zum anderen kommt der Stent beim Platzierungsvorgang mit der distalen Seite des konisch geformten Endes des Stentträgers in Kontakt, sodass der Stent zusammen mit dem Stentträger aus dem äußeren Tubus in distaler Richtung hinausgeschoben werden kann.

In einer besonders vorteilhaften Ausführungsvariante weist der Stentträger an seinem distalen Ende eine radioparke Spitze auf. So kann der Stent unter Röntgenkontrolle in dem Gefäß oder Hohlorgan des Patienten platziert werden. Für den Operateur ist mittels der radioparken Spitze jederzeit ersichtlich, an welcher Stelle im Körper des Patienten sich der Stentträger und somit der Stent befindet.

Bevorzugt entspricht der Außendurchmesser des Stentträgers an seinem proximalen Ende im Wesentlichen dem kleinsten Innendurchmesser der Transportkartusche. Insbesondere entspricht der Außendurchmesser der radiopaken Spitze im Wesentlichen dem kleinsten Innendurchmesser der Transportkartusche. Im Wesentlichen bedeutet im Rahmen der Erfindung, dass das proximale Ende des Stentträgers bzw. die radiopake Spitze, soweit es die Materialpaarung zulässt, bündig mit der Innenseite der Transportkartusche abschließt. Dieses Merkmal ist wichtig für die Überführung des Stentträgers samt Stent aus der Transportkartusche in den äußeren Tubus hinein. Schließt der Stentträger mit seinem proximalen Ende nahezu bündig mit der Innenseite der Transportkartusche ab, wird durch das Einziehen des inneren Tubus, der mit dem Stentträger gekoppelt ist, auch der auf dem Stentträger angeordnete Stent in den äußeren Tubus hineingezogen, ohne von Stentträger zu rutschen.

Vorzugsweise weist der distale Handgriff ein Feststellelement auf, wobei das Feststellelement dazu eingerichtet und ausgebildet ist, den inneren Tubus gegenüber dem äußeren Tubus zu fixieren. Eine entsprechende Fixierung ist insbesondere während des Einführvorgangs des äußeren und inneren Tubus in den Patienten erforderlich. Ein Verrutschen des äußeren Tubus gegenüber dem inneren Tubus während dieses Vorgangs kann erhebliche Komplikationen nach sich ziehen.

Das Feststellelement ist bevorzugt als Schraubverbindung ausgebildet und umfasst eine Mutter. Durch ein Festziehen der Mutter können der innere Tubus und der äußere Tubus gegeneinander fixiert werden, so dass ein Verrutschen verhindert ist. Dabei handelt es sich um einen möglichst simplen Feststellmechanismus, welcher vom Operateur leicht ausführbar ist.

Der Distale Handgriff kann einen Injektionsport aufweisen.

Der proximale Handgriff weist bevorzugt eine Öffnung zum Durchlass eines Führungsdrahts auf.

Die Erfindung kann weiterhin ein Verfahren zum Laden eines Stents, insbesondere eines selbstexpandierenden Stents, in einen Applikator mit den folgenden Verfahrensschritten umfassen:
a) Bereitstellen eines Applikators und eines Stents, wobei ein Applikator verwendet wird, der einen äußeren Tubus und einen inneren Tubus aufweist und dessen innerer Tubus innerhalb des äußeren Tubus geführt ist, wobei der innere Tubus an seinem distalen Ende ein erstes Kopplungselement aufweist, wobei ein Stent verwendet wird, der auf einem Stentträger in einer Transportkartusche angeordnet ist und der Stentträger an seinem proximalen Ende ein zweites Kopplungselement aufweist;
b) Kopplung des ersten Kopplungselements des Applikators mit dem zweiten Kopplungselement des Stentträgers;
c) Verlagerung des inneren Tubus relativ zum äußeren Tubus in proximale Richtung, bis der Stentträger samt Stent aus der Transportkartusche herausgezogen ist und sich vollumfänglich innerhalb des äußeren Tubus befindet.

Die Erfindung wird nachfolgend anhand eines in den Zeichnungen rein schematisch dargestellten Ausführungsbeispiels erläutert. Es zeigen:
- Figur 1: eine erfindungsgemäße Vorrichtung in einem Ausgangszustand;
- Figur 2: die erfindungsgemäße Vorrichtung unmittelbar vor einem gekoppelten Zustand;
- Figur 3: die erfindungsgemäße Vorrichtung im gekoppelten Zustand und
- Figur 4: die erfindungsgemäße Vorrichtung in einem geladenen Zustand.

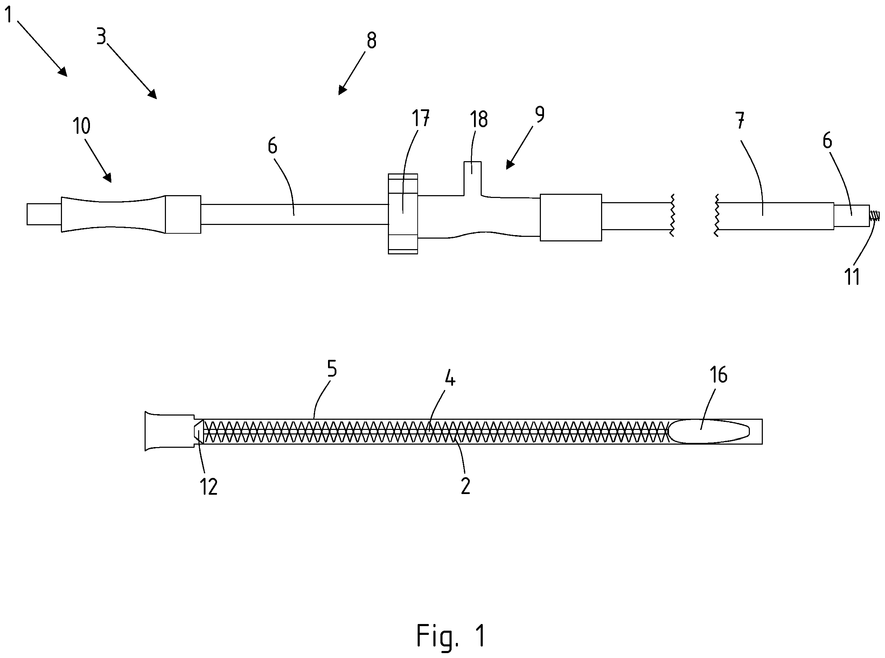

Die Figur 1 zeigt eine erfindungsgemäße Vorrichtung 1 zum Platzieren eines Stents 2 in einem nicht näher dargestellten Gefäß oder einem Hohlorgan eines Menschen oder Tiers in einem Ausgangszustand. Die Vorrichtung 1 weist einen Applikator 3 und einen Stent 2 auf, wobei der Stent 2 auf einem Stentträger 4 in einer Transportkartusche 5 angeordnet ist.

Der Stent 2 besteht aus Nitinol, ist insbesondere in radialer Richtung selbstexpandierend und weist eine Beschichtung auf. Der Stent 2 ist in einem komprimierten Zustand innerhalb der Transportkartusche 5 angeordnet.

Der Stent 2 ist in dem in Figur 1 gezeigten Ausgangszustand nicht im Applikator 3 geladen. Es ist somit möglich, den Stent 2 samt Stentträger 4 und Transportkartusche 5 separat vom Applikator 3 zu sterilisieren. Müssen die Geometrie, die Webart oder die Rückstellkraft des Stents 2 an die speziellen Bedürfnisse eines Patienten angepasst werden, kann einem Operateur ein entsprechend angepasster Stent 2, der anstelle eines Niedrigtemperatursterilisationsverfahrens mit einem Dampfsterilisationsverfahren direkt vom Stenthersteller sterilisiert werden kann, nebst einem vorab niedrigtemperatursterilisiertem Applikator 3 zur Verfügung gestellt werden. Eine Sterilisation des Stents 2 von einem speziell zugelassenem Sterilisationsdienstleister ist somit nicht erforderlich. Dies spart Kosten und Zeit.

Der Applikator 3 weist einen inneren Tubus 6 und einen äußeren Tubus 7 auf. Der innere Tubus 6 ist innerhalb des äußeren Tubus 7 geführt.

Der Applikator 2 weist an seinem proximalen Ende 8 einen distalen Handgriff 9 und einen proximalen Handgriff 10 auf. Der distale Handgriff 9 ist mit einem proximalen Ende des äußeren Tubus 7 und der proximale Handgriff 10 mit einem proximalen Ende des inneren Tubus 6 gekoppelt. Mittels der Handgriffe 9, 10 ist der inneren Tubus 6 gegenüber dem äußeren Tubus 7 verlagerbar.

Der innere Tubus 6 weist an seinem distalen Ende ein erstes Kopplungselement 11 auf. Der Stentträger 4 weist an seinem proximalen Ende ein zweites Kopplungselement 12 auf. Das erste Kopplungselement 11 ist mit dem zweiten Kopplungselement 12 koppelbar. Die Kopplung des Stentträgers 4 mit dem inneren Tubus 6 des Applikators 3 ist für den Operateur der erste Schritt, um den Stent 2 in den Applikator 3 zu laden, mithin diesen in den äußeren Tubus 7 des Applikators 3 zu überführen.

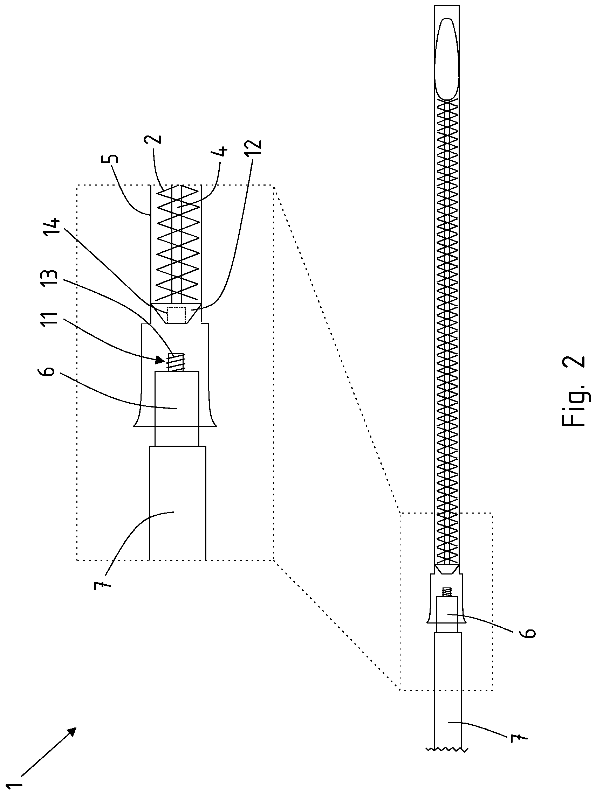

Die Figur 2 zeigt die Vorrichtung 1 unmittelbar vor der Kopplung. Das erste Kopplungselement 11 des Applikators 3 weist ein Außengewinde 13 auf, während das zweite Kopplungselement 12 des Stentträgers 4 ein Innengewinde 14 aufweist. So können der innere Tubus 6 und der Stentträger 4 mittels einer Schraubverbindung gekoppelt werden.

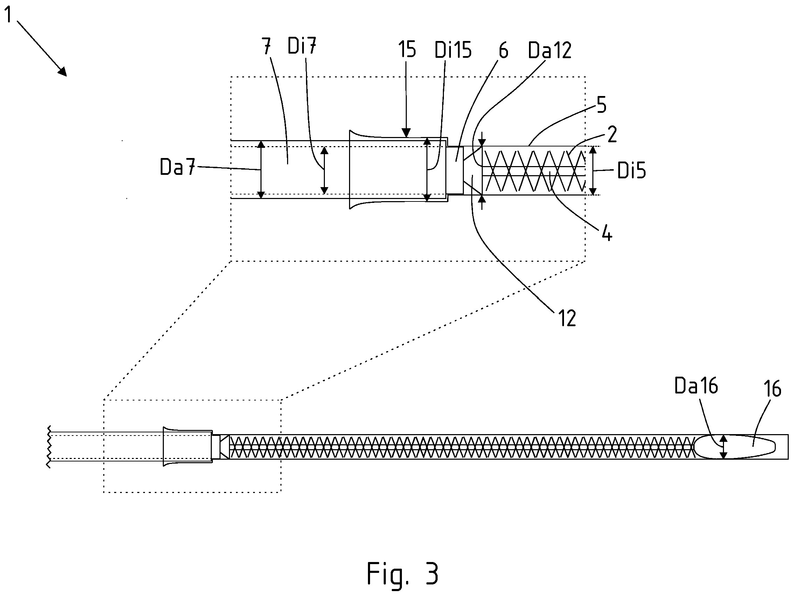

Die Figur 3 zeigt die Vorrichtung 1 im gekoppelten Zustand der Kopplungselemente 11, 12. Die Transportkartusche 5 ist an ihrem proximalen Ende aufgeweitet. Nach dem Kopplungsvorgang kann der äußere Tubus 7 in distaler Richtung in den aufgeweiteten Bereich 15 der Transportkartusche 5 hineingeschoben werden. Um dies zu ermöglichen ist der Innendurchmesser Di15 der Transportkartusche 5 im aufgeweiteten Bereich 15 größer als der Außendurchmesser Da7 des äußeren Tubus 7.

Zudem ist der Innendurchmesser Di5 des an dem aufgeweiteten Bereich 15 angrenzenden Bereich der Transportkartusche 5 kleiner als der Außendurchmesser Da7 des äußeren Tubus 7. Der äußere Tubus 7 kommt somit am Übergang zwischen dem aufgeweiteten Bereich 15 und dem übrigen Bereich der Transportkartusche 5 mit dieser zur Anlage. Anschließend wird der innere Tubus 6 zusammen mit dem an diesem gekoppelten Stentträger 4 und dem darauf angeordneten Stent 2 in proximaler Richtung in den äußeren Tubus 7 hineingezogen.

Die Transportkartusche 5 ist an ihren jeweiligen Enden geöffnet. Die Öffnung am proximalen Ende der Transportkartusche 5 ermöglicht die Kopplung mit dem Applikator 3, während die Öffnung am distalen Ende der Transportkartusche 5 dafür sorgt, dass während des Einziehvorgangs kein Vakuum bzw. Unterdruck in der Transportkartusche 5 gebildet wird, welcher die Überführung in den Applikator 3 erschweren würde.

Der Stentträger 4 weist an seinem distalen Ende eine radiopake Spitze 16 auf. So kann der Stent 2 unter Röntgenkontrolle in dem Gefäß oder Hohlorgan des Patienten platziert werden. Für den Operateur ist mittels der radiopaken Spitze 16 jederzeit ersichtlich, an welcher Stelle im Körper des Patienten sich der Stentträger 4 und somit der Stent 2 befindet.

Der maximale Außendurchmesser Da16 der radiopaken Spitze 16 entspricht im Wesentlichen dem kleinsten Innendurchmesser Di5 der Transportkartusche 5 und dem Innendurchmesser Di7 des äußeren Tubus 7. Dieses Merkmal ist wichtig für die Überführung des Stentträgers 4 samt Stent 2 aus der Transportkartusche 5 in den äußeren Tubus 7 hinein. Schließt die radiopake Spitze 16 im Wesentlichen bündig mit der Innenseite der Transportkartusche 5 ab, wird durch das Einziehen des inneren Tubus 6, der mit dem Stentträger 4 gekoppelt ist, in proximale Richtung auch der auf dem Stentträger 4 angeordnete Stent 2 in den äußeren Tubus 7 hineingezogen. Ein lösen des Stents 2 vom Stentträger 4 wird verhindert.

Das zweite Kopplungselement 12 des Stentträgers 4 ist konisch geformt. Der Durchmesser Da12 des zweiten Kopplungselements 12 reduziert sich in proximaler Richtung. Dies ermöglicht ein einfaches Einziehen des Stentträgers 4 in den äußeren Tubus 7 ohne ein Verkanten.

Der maximale Außendurchmesser Da12 des zweiten Kopplungselements 12 entspricht im Wesentlichen dem Innendurchmesser Di7 des äußeren Tubus 7 und dem minimalen Innendurchmesser Di5 der Transportkartusche 5. Dieses Merkmal ist wichtig für das eigentliche Platzieren bzw. Entladen des Stents 2, da der Stent 2 durch das zweite Kopplungselement 12 des Stentträgers 4 aus dem äußeren Tubus 7 in distale Richtung hinausgeschoben wird, oder der äußere Tubus 7 relativ zum inneren Tubus 6 in proximaler Richtung verlagert wird, sodass der Stent 2 zur Expansion im Gefäß oder Hohlorgan freigeben wird.

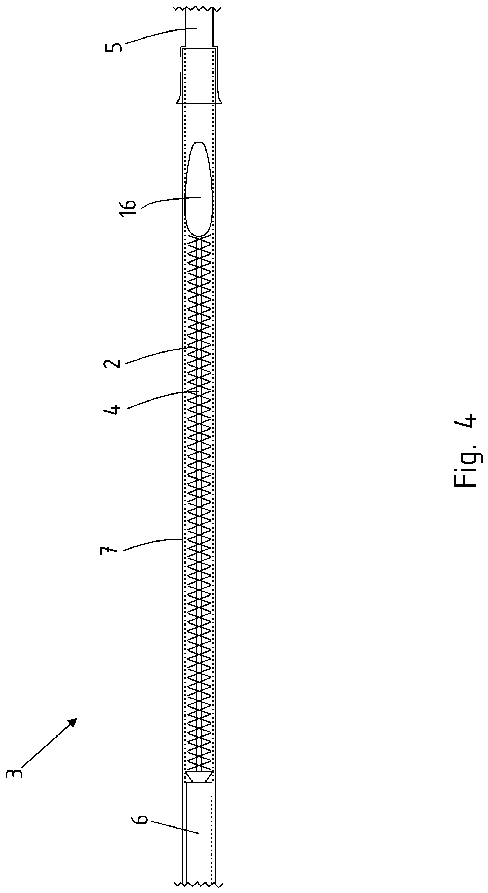

Die Figur 4 zeigt den Applikator 3 im geladenen Zustand. Der Stent 2 ist vollständig im äußeren Tubus 7 angeordnet. Die Transportkartusche 5 kann nach dem Ladevorgang vom äußeren Tubus 7 abgezogen und entsorgt werden.

Ist der Stent 2 im Applikator 3 geladen, wird der äußere Tubus 7 gegenüber dem inneren Tubus 6 fixiert, um eine Verlagerung während des Platzierend des Stents 2 im Patienten zu verhindern. Wie in der Figur 1 zu sehen ist weist der distale Handgriff 9 dafür ein Feststellelement 17 auf. Mittels des Feststellelements 17, welches als Schraubverbindung ausgebildet ist und eine Mutter umfasst, kann der äußere Tubus 7 gegenüber dem inneren Tubus 6 fixiert werden. So wird ein Verrutschen des äußeren Tubus 7 gegenüber dem inneren Tubus 6 verhindert. In diesem Zustand kann der Stent 2 mittels des Applikators 3 im Patienten platziert werden.

Der distale Handgriff 9 weist zudem einen Injektionsport 18 auf.

### Bezugszeichen:

- 1 -: Vorrichtung
- 2 -: Stent
- 3 -: Applikator
- 4 -: Stentträger
- 5 -: Transportkartusche
- 6 -: innerer Tubus
- 7 -: äußerer Tubus
- 8 -: distales Ende von 3
- 9 -: distaler Handgriff
- 10 -: proximaler Handgriff
- 11 -: erstes Kopplungselement
- 12 -: zweites Kopplungselement
- 13 -: Außengewinde
- 14 -: Innengewinde
- 15 -: aufgeweiteter Bereich von 5
- 16 -: radiopake Spitze
- 17 -: Feststellelement
- 18 -: Injektionsport

- Di7 -: Innendurchmesser von 7
- Da7 -: Außendurchmesser von 7
- Di15 -: Innendurchmesser von 15
- Di5 -: Innendurchmesser von 5
- Da12 -: Außendurchmesser von 12
- Da16 -: Außendurchmesser von 16

## Patentansprüche

1. Vorrichtung (1) zum Platzieren eines Stents (2) in einem Gefäß oder einem Hohlorgan eines Menschen oder Tiers, aufweisend einen Applikator (3) und einen Stent (2) mit den folgenden Merkmalen:
a) Der Applikator (3) weist einen äußeren Tubus (7) und einen inneren Tubus (6) auf, wobei der innere Tubus (6) innerhalb des äußeren Tubus (7) geführt ist;
b) Der Stent (2) ist auf einem Stentträger (4) in einer Transportkartusche (5) angeordnet;
c) Der innere Tubus (6) weist an seinem distalen Ende ein erstes Kopplungselement (11) auf;
d) Das erste Kopplungselement (11) des Applikators (3) ist mit einem zweiten Kopplungselement (12) am proximalen Ende des Stentträgers (4) koppelbar;
e) Der Stentträger (4) ist im gekoppelten Zustand der Kopplungselemente (11,12) zusammen mit dem Stent (2) aus der Transportkartusche (5) heraus und in den äußeren Tubus (7) einziehbar um den Stent (2) innerhalb des äußeren Tubus (7) anzuordnen.

2. Vorrichtung (1) nach Anspruch 1, **dadurch gekennzeichnet, dass** das erste Kopplungselement (11) oder das zweite Kopplungselement (12) ein Außengewinde (13) aufweist, wobei das jeweils andere Kopplungselement (11, 12) ein Innengewinde (14) aufweist.

3. Vorrichtung (1) nach Anspruch 1 oder 2, **dadurch gekennzeichnet, dass** der Applikator (3) an seinem proximalen Ende (8) einen distalen Handgriff (9) und einen proximalen Handgriff (10) aufweist, wobei der distale Handgriff (9) mit einem proximalen Ende des äußeren Tubus (7) und der proximale Handgriff (10) mit einem proximalen Ende des inneren Tubus (6) gekoppelt ist.

4. Vorrichtung (1) nach einem der vorstehenden Ansprüche, **dadurch gekennzeichnet, dass** die Transportkartusche (5) an ihrem proximalen Ende aufgeweitet ist.

5. Vorrichtung (1) nach einem der vorstehenden Ansprüche, **dadurch gekennzeichnet, dass** der Innendurchmesser (Di15) der Transportkartusche (5) im aufgeweiteten Bereich (15) größer ist als der Außendurchmesser (Da7) des äußeren Tubus (7), wobei zumindest der Innendurchmesser (Di5) des an dem aufgeweiteten Bereich (15) angrenzenden Bereichs der Transportkartusche (5) kleiner ist als der Außendurchmesser (Da7) des äußeren Tubus (7).

6. Vorrichtung (1) nach einem der vorstehenden Ansprüche, **dadurch gekennzeichnet, dass** die Transportkartusche (5) an ihren jeweiligen Enden geöffnet ist.

7. Vorrichtung (1) nach einem der vorstehenden Ansprüche, **dadurch gekennzeichnet, dass** der Stentträger (4) an seinem proximalen Ende konisch geformt ist.

8. Vorrichtung (1) nach einem der vorstehenden Ansprüche, **dadurch gekennzeichnet, dass** der Außendurchmesser (Da12) des Stentträgers (4) an seinem proximalen Ende im Wesentlichen dem Innendurchmesser (Di7) des äußeren Tubus (7) entspricht.

9. Vorrichtung (1) nach einem der vorstehenden Ansprüche, **dadurch gekennzeichnet, dass** der Stentträger (4) an seinem distalen Ende eine radiopake Spitze (16) aufweist.

10. Vorrichtung (1) nach einem der vorstehenden Ansprüche, **dadurch gekennzeichnet, dass** der distale Handgriff (9) ein Feststellelement (17) aufweist, wobei das Feststellelement (17) dazu eingerichtet und ausgebildet ist den inneren Tubus (6) gegenüber dem äußeren Tubus (7) zu fixieren.

11. Vorrichtung (1) nach einem der vorstehenden Ansprüche, **dadurch gekennzeichnet, dass** das Feststellelement (17) als Schraubverbindung ausgebildet ist und eine Mutter umfasst.

12. Vorrichtung (1) nach einem der vorstehenden Ansprüche, **dadurch gekennzeichnet, dass** der Stent (2) aus Nitinol besteht und selbstexpandierend ist.

13. Vorrichtung (1) nach einem der vorstehenden Ansprüche, **dadurch gekennzeichnet, dass** der Stent (2) eine Beschichtung aufweist.
